Europäisches Patentamt

European Patent Office

Office européen des brevets

(11) Publication number: **0 108 612**
**B1**

## (12) EUROPEAN PATENT SPECIFICATION

(45) Date of publication of patent specification: **08.04.87**

(51) Int. Cl.⁴: **G 01 N 33/569**

(21) Application number: **83306694.7**

(22) Date of filing: **03.11.83**

(54) Composition and method for detecting antistreptolysin O.

(30) Priority: **08.11.82 US 440049**

(43) Date of publication of application:
**16.05.84 Bulletin 84/20**

(45) Publication of the grant of the patent:
**08.04.87 Bulletin 87/15**

(84) Designated Contracting States:
**AT BE CH DE FR GB IT LI LU NL SE**

(56) References cited:
**US-A-4 148 609**
**US-A-4 379 850**

(73) Proprietor: **AMERICAN HOME PRODUCTS CORPORATION**
**685, Third Avenue**
**New York, New York 10017 (US)**

(72) Inventor: **Green, Lorrence Howell**
**17 Pride Lane**
**Westbury New York 11590 (US)**
Inventor: **Roberts, John Walter**
**Mary Road**
**Milford Massachusetts 01757 (US)**

(74) Representative: **Brown, Keith John Symons et al**
**c/o John Wyeth & Brother Limited Patent and Trademark Department Huntercombe Lane South**
**Taplow Maidenhead Berkshire SL6 0PH. (GB)**

Courier Press, Leamington Spa, England.

## Description

Streptolysin 0 (hereinafter also referred to as "SO") is a hemolytic protein produced by most group A streptococci. One property of SO is its antigenicity with the result that antistreptolysin O (hereinafter also referred to as "ASO") is usually produced in response to Streptococcus A infections. While ASO binds SO and inhibits SO hemolytic activity, the amount of ASO produced in response to the infection may be in excess of the amount required to inhibit the SO. Moreover, repeat infections frequently trigger an amplified immune response and excess production of ASO. Because ASO also bonds to M type proteins, this leads to secondary immune responses, the sequellae of which include degeneration or destruction of certain heart and kidney tissues which may result in rheumatic fever or acute glomerulonephritis.

It is also well known that SO hemolytic activity is neutralized or destroyed in its oxidized state. Since the SO antigen is readily available commercially and because of the described properties of SO and ASO, the measurement and monitoring of a patient ASO titer has been a feasible and useful diagnostic tool.

Most commercially available kits for determining ASO titer are based on the Rantz and Randall methodology Proc. Soc. Exp. Biol. Med. (N.Y.) 59, 22 (1945). The procedure calls for the technician to draw several mls of blood, and then process it into serum. The serum is then diluted and incubated at 37°C with SO. After an initial incubation period of 15 minutes, a constant amount of 5% washed rabbit, sheep, or human type O red blood cells (RBC's) are added, and the samples are again incubated. ASO titer is determined about 45 minutes later by noting which tubes contain lysed RBC's. The procedure is very time consuming and tedious. It also has several steps at which pipetting errors can easily occur and cause inaccurate results. In addition, it requires that the technician have access to a centrifuge, an incubator, and a fresh supply of washed 5% RBC's.

A more recent procedure is described by Ricci *et al*, J. Clin. Microb., Vol. 8, No. 3, 263—267 (1978) and in US Patent 4,148,609 issued April 10, 1979. In the Ricci method SO is chemically altered through oxidation to form disulfide bonds, chemically inactivating the hemolytic activities of the molecules. After incubating the oxidized SO with a blood sample possibly containing ASO, the SO is once again chemically altered with a reducing substance to reactivate the SO hemolytic activity. The SO in excess of that necessary to bind all the available ASO is then free to lyse the erythrocytes in the blood sample.

In most of these tests for determining ASO, the measurements are expressed in Todd Units. Thus one Todd Unit (TU) of SO has been arbitrarily defined as the amount of SO needed to completely lyse 1 ml of 5% red blood cells in saline in one hour at 37°C. Correlatively one TU of ASO has been defined as that amount of ASO which bonds to $2\frac{1}{2}$ TU of SO. Whether one uses Todd Units or any other unit, arbitary or otherwise, correlating the hemolytic activity of SO with its ability to bind with ASO, however, is a matter of choice, so long as such expressions or measurements are related to the observed ASO values of normal diseased patients.

In this disclosure the term "hemolytic activity" has been used and is defined as the ability of SO to lyse blood cells; and the term "hemolytic capacity" has been used and is defined in relation to the amount of hemolytic activity and correlatively the amount of binding ability to ASO possessed by SO.

This invention relates to compositions of Streptolysin O (SO) which are useful as reagents in measuring or determining, either qualitatively or quantitatively, the antistreptolysin O (ASO) titer of an unknown sample of blood, e.g. human blood. The present invention provides a reagent composition containing reversibly hemolytically inactive Streptolysin O, for use in measuring the amount of Antistreptolysin O in a sample, which composition comprises a solution of Streptolysin O and an acid, base or buffer in an amount sufficient to maintain the pH of the composition greater than 9.0 or less than 4.8. Advantageously the composition of the invention is employed in a dry form which can be achieved by lyophilizing, vacuumdrying or other methods known in the art. Accordingly the invention also provides a dry reagent composition containing reversibly hemolytically inactive Streptolysin O, for use in measuring the amount of Antistreptolysin O in a sample, which comprises Streptolysin O in dry form and an acid, base or buffer in an amount sufficient to maintain the pH of the composition greater than 9.0 or less than 4.8.

The advantages of the present invention stem from the discovery that the hemolytic activity of SO is sensitive to pH. Thus it has been found that the hemolytic activity of SO can be reversibly neutralized or inactivated by adjusting the pH of the composition outside the hemolytic activity range of SO (i.e. outside the range 4.8 to 9.0) without substantially affecting or reducing the hemolytic capacity of the SO so treated. As a result when the pH of an SO containing composition is readjusted to within the hemolytic activity range of SO, the hemolytic capacity of the SO in the composition is restored or reactivated.

Another embodiment of this invention relates to a method for measuring the ASO titer or concentration in a sample of blood whether whole blood or the serum thereof. Broadly stated, this method involves, first, mixing at least one sample of non-hemolyzed blood with at least one predetermined quantity of SO. The mixing is achieved in a non-hemolytic solution, the pH of which is outside the hemolytic activity range of SO and the SO employed in the method has a known hemolytic capacity.

In a second step each solution is incubated for a time sufficient to allow any ASO in the blood sample to react (or bind) with the SO. The hemolytic activity of the unbound SO is then restored (or reactivated) by adjusting the pH of each incubated solution to a range within the hemolytic activity range of SO, whereby any free (or unbound) SO in excess of the amount required to react with the ASO can lyse blood, i.e. the blood cells. The final step of the method involves detecting the presence or absence of lysis in each solution.

As mentioned above, in one aspect, this invention relates to a composition comprising Streptolysin O (SO) at a pH outside the hemolytic activity range of SO (i.e. outside a pH of from 4.8 to 9.0 and preferably from 4.6 to 9.5—9.6). The usefulness of the composition of the invention, whether in solution or dried form, arises from the discovery that the hemolytic activity of SO is reversibly sensitive to pH. Accordingly the composition can be employed as a reagent for the detection or measurement of Antistreptolysin O (ASO) or for monitoring the course of a disease state or infection or the effectiveness of a course of treatment against a disease state or infection which produces ASO antibody in response to a group A streptococcus infection.

While not wishing to be bound by any theory of invention, it is believed that hemolytic activity is optimized at certain pH ranges and as the pH is adjusted outside such optimum ranges, the hemolytic activity undergoes a progressive pH induced change in the conformation of the (hemolytically) active site of SO. Also, while the adjustment of the pH to a pH outside the hemolytic activity range of SO can totally inhibit SO hemolytic activity, such adjustment does not affect SO binding or reaction sites to ASO or otherwise destroy or permanently inactivate the hemolytic capacity of the SO so treated.

Another advantage of the composition and method of the invention is that the use of the invention does not require separation of the serum from blood or stripping the blood serum from its complements. While the method of the invention allows the use of such separated fractions, the method may be most advantageously conducted on whole blood using even small samples thereof.

The composition of the invention employs an otherwise hemolytically active form of SO, the activity of which has been reversibly inactivated by the previously described pH adjustment to a pH outside the hemolytic activity range of SO. More particularly the composition and method of the invention utilize the hemolytically active, reduced form of SO.

While there are many routes to obtaining the hemolytically active form of SO, the following embodiment illustrates a procedure that was advantageously employed. A hemolytic strain of Streptococcus pyogenes Type I (Group A), which had been stored frozen, was thawed, streaked on an agar plate containing 5% blood and incubated at 37°C for 18—24 hours. Colonies from this first plate were streaked and incubated on a second plate under the same conditions. Several colonies from the second plate were then used to inoculate 10 ml of Todd-Hewitt (TH) broth which was then incubated at 37°C for 18—24 hours. Following incubation 1.0 ml of the first TH suspension was inoculated into a second tube containing 10 ml of TH broth and incubated and then 1 ml from the second tube is inoculated into a third container having 100 ml of TH broth. The one hundred millilitres of the last incubated suspension was then inoculated into 10 litres of TH broth, incubated at 37°C and then 25°C until all the bacteria settled, after which the bacteria was removed by filtration. The filtrate containing SO may be stored frozen after adjusting the pH to 6.75. Prior to use, the SO in the filtrate is reduced to restore and preserve SO hemolytic activity which may have been lost in the preparation of the SO through oxidation. Cysteine is the preferred reducing agent although other reducing agents known in the art for reducing SO may be used.

According to one embodiment of conducting the method of the invention one or more predetermined quantities of the hemolytically active form of SO may be mixed with at least one sample of non-hemolyzed blood in a non-hemolytic solution having a pH outside the hemolytic activity range of SO. Alternatively, the pH of the filtrate or solution containing the hemolytically active SO is adjusted to a pH outside the hemolytic activity range of SO to provide the composition of the invention. Preferably the pH is adjusted to 9.4 or more preferably to 9.5—9.6 or higher, with a basic substance such as sodium or potassium hydroxide and/or buffer. While the pH adjusted (inactivated) SO may be stored frozen, it is preferable to immediately employ said SO in an appropriate dilution or dilutions, by mixing with at least one sample of non-hemolyzed blood whereby the pH of the mixed solution remains outside the hemolytic activity range of SO in another embodiment of conducting the method of the invention. Most preferably, the filtrate (solution) containing the hemolytically active (reduced) SO is vacuum dried either, in predetermined quantities or dilutions, directly in a test chamber or chambers, or, dried and then placed in the test chamber or chambers. The composition of the invention in dry form is stable at room temperature but storage under refrigerated conditions provides longer stability. As noted elsewhere, the hemolytically active SO employed in the invention will have a known or predetermined hemolytic capacity.

Thus, the invention provides a method for measuring the Antistreptolysin O (ASO) titer in a sample of blood or serum comprising the steps of

(a) mixing at least one sample of non-hemolyzed blood or serum with at least one predetermined quantity of Streptolysin O (SO) in a non-hemolytic solution having a pH outside the hemolytic activity range of SO (i.e. outside the range of 4.8 to 9.0), said SO having a known hemolytic capacity;

(b) incubating each solution, e.g. at a temperature of 0—50°C, for a time sufficient to allow any ASO in the blood or serum sample to react with the SO;

(c) restoring the hemolytic activity of the SO by adjusting the pH of each incubated solution to within the hemolytic activity range of SO (i.e. a pH of 4.8 to 9.0), whereby any free SO in excess of the amount required to react with the ASO can lyse blood cells present in the sample; and

(d) detecting the presence or absence of lysis in each solution.

The method of this invention for measuring ASO titer is quite flexible and lends itself to various advantageous pathways. For example, the composition of the invention may be employed either directly in solution or dry form, or indirectly through the introduction and mixing of SO into a non-hemolytic solution at a pH outside the hemolytic activity range of SO with or without the blood sample. Similarly the SO in solution or dry form, may be added to the blood sample or the blood sample may be added to the SO. The method is further adaptable to using equal or graduated volume(s) or amount(s) of the blood sample and to using equal or graduated concentrations of the SO in liquid or dry form. The method of the invention as described is also adaptable to the inactivation or inhibition of the hemolytic activity either above or below the pH range within which SO possesses hemolytic activity. However, the invention is most advantageously conducted when the inactivation results from raising or maintaining the SO pH, i.e. to a pH greater than 9, preferably to a pH greater than 9.4 and most preferably to a pH greater than 9.5—9.6. On the acid side the analogous progression to the most preferred pH is less than 4.8 and preferably less than 4.6.

After mixing a particular amount or dilution of the blood or serum sample with the SO, the solution formed is incubated for a time sufficient to allow any ASO in the sample to react or bind with the SO. In this invention a time of about 1 to about 10 minutes has proved sufficient, although longer incubation periods and a time as short as one minute are acceptable. The incubation temperature may range from 0°C to 50°C, but is preferably from room temperature (18—21°C) to 50°C and most preferably 20—25°C.

Depending on which end of the pH range has been employed to inhibit or reversibly inactivate the SO hemolytic activity, either an acidic or basic substance, preferably a buffered substance will be used to restore the SO hemolytic activity by adjusting the pH of the incubated solution within the hemolytic activity range. The step of restoring hemolytic activity may also be advantageously applied employing a reducing substance such as cysteine together with the buffer substance or with a reducing buffer per se. In this way, prophylaxis towards oxidation would not be a concern nor would the use of an initially reduced form of SO be required. Other buffers which may be employed include sodium and/or potassium sulfite, phosphate, thiosulfate or thiosulfite, imidazole, citrate and 2-N-morpholino ethane sulfonate.

Accordingly while the pH may be adjusted within the range of 4.8 to 9.0, full hemolytic capacity is best restored within the range of 6 to 8, and most particularly within the range of 6.5 to 7.0. Once the activity is restored, excess SO not bound or reacted with the ASO present in the sample will proceed to lyse the blood cells also present in the sample.

While lysis of the cells will immediately begin once hemolytic activity is restored it is best to wait or incubate the solutions at least 1—2 to 10 minutes before detecting the presence or absence of lysis in each solution. Generally, the recommended time to wait or incubate before detection will depend on whether a qualitative or quantitative test for ASO is desired. In the former case, typically, only one blood sample is being tested against one quantity of SO and accordingly less time is efficacious. In the latter case more than one test is studied at one time and accordingly the incubation time prior to detection should preferably range over at least 5 to 10 minutes.

Conveniently, lysis can be detected immediately after incubation. To avoid false or misleading results, however, it is advisable to detect the presence or absence of lysis within 20 minutes after incubation restoring SO hemolytic activity and preferably within 10 or 15 minutes. In sum detection of the presence or absence of lysis will normally be made within 1 to 30 minutes following the pH adjustment restoring SO hemolytic activity and the preferred times will range over the various sums of the waiting (incubation) and detection times indicated.

The presence of lysis will result in a clear solution and the absence of lysis or incomplete lysis will be indicated by the presence of turbidity in the solution. For practical purposes, positive and negative controls are desirable but not necessary.

As described, the method of this invention for measuring the ASO titer in a sample of blood is most conveniently and preferably conducted with a small sample of whole blood which is readily obtained, for example, by ear, heel or finger puncture. Anticoagulants or anticoagulated blood are not required for the test when the blood sample is immediately diluted for use. For example 40 microlitres of the blood may be diluted with 2.0 ml of physiological saline and a volume or equal volumes of the diluted blood are introduced into one or a series of test chambers. Aside from any controls, each test chamber contains graduated amounts of the composition of this invention. The sample is mixed with the SO in each test chamber and the solution thus formed is incubated. The pH of each incubated solution is then adjusted, e.g. within the range of 6 to 8 and the presence or absence of lysis is detected.

A method of monitoring the course of a disease state or infection arising from a group A Streptococcus infection according to the invention comprises

(1) measuring the antistreptolysin O (ASO) titer in blood samples taken from the same subject at different times during the course of the disease state or infection by a method described hereinabove, and

(2) determining from successive measurements changes in the ASO titer from the subject; whereby measured increases in the ASO titer indicates progression of the disease state or infection, and whereby measured decreases in the ASO titer indicates regression or cure of the disease state or infection.

**Claims**

1. A reagent composition containing reversibly hemolytically inactive Streptolysin O, for use in measuring the amount of Antistreptolysin O in a sample, which composition comprises a solution of Streptolysin O and an acid, base or buffer in an amount sufficient to maintain the pH of the composition greater than 9.0 or less than 4.8.

2. A composition as claimed in Claim 1 having a pH greater than 9.5 or less than 4.6.

3. A composition as claimed in Claim 1 wherein the pH is at least 9.5.

4. A dry reagent composition containing reversibly hemolytically inactive Streptolysin O for use in measuring the amount of Antistreptolysin O in a sample, which comprises Streptolysin O in dry form and an acid, base or buffer in an amount sufficient to maintain the pH of the composition greater than 9.0 or less than 4.8, once the dry composition is reconstituted with water.

5. A composition as claimed in Claim 4 wherein the amount of the base or buffer is sufficient to maintain the pH of the composition of at least 9.5.

6. A method for preparing a reversibly hemolytically inactive Streptolysin O reagent for use in measuring the amount of Antistreptolysin O in a sample, which comprises mixing Streptolysin O with an acid, base or buffer in amount sufficient to maintain the pH greater than 9.0 or less than 4.8.

7. A method as claimed in Claim 6 in which the mixture is dried to give the reagent in dried form.

8. A method for measuring the Antistreptolysin O (ASO) titer in a sample of blood or serum comprising the steps of

(a) mixing at least one sample of non-hemolyzed blood or serum with at least one predetermined quantity of Streptolysin O (SO) in a non-hemolytic solution having a pH greater than 9.0 or less than 4.8, said SO having a known hemolytic capacity;

(b) incubating each solution for a time sufficient to allow any ASO in the sample to react with the SO;

(c) restoring the hemolytic activity of the SO by adjusting the pH of each incubated solution to a pH within the range of 4.8 to 9.0 whereby any free SO in excess of the amount required to react with the ASO can lyse blood cells present in the sample; and

(d) detecting the presence or absence of lysis in each solution.

9. A method as claimed in Claim 8 wherein in step (b) each solution is incubated at a temperature of 0 to 50°C.

10. A method as claimed in Claim 8 or 9 in which step (a) further comprises

(1) preparing at least one solution of SO having a pH outside the range of 4.8 to 9.0; and mixing a volume of each SO solution with a blood or serum sample, whereby the pH of the mixed solution remains outside the range of 4.8 to 9.0; or

(2) preparing at least one dried sample of SO, the SO having a pH in solution outside the range 4.8 to 9.0; and mixing each sample of SO with a non-hemolyzed blood or serum sample in a non-hemolytic solution, whereby the pH of the mixed solution remains outside the range of 4.8 to 9.0; or

(3) diluting the blood or serum sample in a non-hemolytic solution; introducing equal volumes or graduated volumes of the blood or serum sample solution into each of a series of test chambers; and adding and mixing into each of the test chambers containing the blood or serum samples equal concentrations or graduated concentrations of SO hemolytically inactivated by pH whereby the pH of the mixed solution in each test chamber remains outside the range of 4.8 to 9.0; or

(4) introducing equal concentrations or graduated concentrations or dried SO hemolytically inactivated by pH into each of a series of test chambers; diluting the blood or serum sample in a non-hemolytic solution; and adding and mixing into each of the test chambers containing the SO equal volumes or graduated volumes of the non-hemolytic blood or serum sample solution, whereby the pH of the mixed solution in each test chamber remains outside the range of 4.8 to 9.0.

11. A method as claimed in any one of Claims 8 to 10 wherein

(a) the mixing of the blood or serum sample with the SO is conducted at a pH of at least 9.4;

(b) each mixed solution is incubated at a temperature in the range between room temperature and 50°C; and

(c) each incubated solution is adjusted to a pH within the range of 6 to 8.

12. A method for measuring the Antistreptolysin O (ASO) titer in a sample of blood or serum comprising the steps of

(a) diluting a non-hemolyzed blood or serum sample with a non-hemolytic solution;

(b) introducing an equal volume of the diluted blood or serum sample into a plurality of test chambers, each of the test chambers containing a dried sample of hemolytically inactivated SO in graduated amounts, the SO having a predetermined hemolytic capacity and providing in solution a pH of at least 9.4;

(c) mixing each sample of diluted blood or serum with the SO;

(d) incubating each mixed solution at a temperature ranging from room temperature to 50°C for 1 to 10 minutes;

(e) adjusting the pH of each incubated solution to a pH ranging from 6 to 8; and

(f) detecting the presence or absence of lysis in each incubated solution 5 to 25 minutes after adjusting the pH of each solution.

13. A method for measuring the Antistreptolysin O (ASO) titer in a sample of blood or serum comprising the steps of

(a) serially diluting a non-hemolyzed blood or serum sample with a non-hemolytic solution;

(b) introducing equal volumes of the serially diluted blood or serum sample into a plurality of test chambers, each of the test chambers containing a dried sample of hemolytically inactivated SO in equal amounts, said SO having a predetermined hemolytic capacity and providing in solution a pH of at least 9.4;

(c) Mixing each sample of the serially diluted blood or serum with the SO;

(d) incubating each mixed solution at a temperature ranging from room temperature to 50°C for 1 to 10 minutes;

(e) adjusting the pH of each incubated solution to a pH ranging from 6 to 8; and

(f) detecting the presence or absence of lysis in each incubated solution 2 to 20 minutes after adjusting the pH of each solution.

14. A method for monitoring the course of a disease state or infection arising from a group A Streptococcus infection comprising

(1) measuring, by a method claimed in any one of Claims 8 and 13, the Antistreptolysin O (ASO) titer in blood samples taken from the same subject at different times during the course of the disease state or infection; and

(2) determining from successive measurements changes in the ASO titer from the subject:

whereby measured increases in the ASO titer indicates progression of said disease state or infection, and

whereby measured decreases in the ASO titer indicates regression or cure of said disease state or infection.

**Patentansprüche**

1. Reagenszusammensetzung, die reversibel hämolytisch inaktives Streptolysin O enthält, zur Verwendung beim Messen des Anteils von Antistreptolysin O in einer Probe, welche Zusammensetzung eine Lösung von Streptolysin O und eine Säure, eine Base oder einen Puffer in einem Anteil umfaßt, der ausreicht, den pH der Zusammensetzung höher als 9,0 oder niedriger als 4,8 zu halten.

2. Zusammensetzung, wie in Anspruch 1 beansprucht, mit einem pH von höher als 9,5 oder niedriger als 4,6.

3. Zusammensetzung, wie in Anspruch 1 beansprucht, worin der pH zumindest 9,5 beträgt.

4. Trockene Reagenszusammensetzung, die reversibel hämolytisch inaktives Streptolysin O enthält, zur Verwendung beim Messen des Anteils von Antistreptolysin O in einer Probe, welche Streptolysin O in trockener Form und eine Säure, eine Base oder einen Puffer in einem Anteil umfaßt, der ausreicht, den pH der Zusammensetzung höher als 9,0 oder niedriger als 4,8 zu halten, sobald die trockene Zusammensetzung mit Wasser rekonstituiert ist.

5. Zusammensetzung, wie in Anspruch 4 beansprucht, worin der Anteil der Base oder des Puffers ausreicht, den pH der Zusammensetzung bei mindestens 9,5 zu halten.

6. Verfahren zum Herstellen von reversibel hämolytisch inaktivem Streptolysin O-Reagens zur Verwendung beim Messen des Anteils von Antistreptolysin O in einer Probe, welches des Mischen von Streptolysin O mit einer Säure, einer Base oder einem Puffer in einem Anteil, der ausreicht, den pH höher als 9,0 oder niedriger als 4,8 zu halten, umfaßt.

7. Verfahren, wie in Anspruch 6 beansprucht, worin die Mischung getrocknet wird, um das Reagens in getrockneter Form zu ergeben.

8. Verfahren zum Messen des Antistreptolysin O (ASO)-Titers in einer Probe von Blut oder Serum, umfassend die Schritte

(a) des Mischens von zumindest einer Probe von nicht-hämolysiertem Blut oder Serum mit zumindest einem vorherbestimmten Anteil von Streptolysin O (SO) in einer nicht-hämolytischen Lösung mit einem pH von höher als 9,0 oder niedriger als 4,8, wobei das SO eine bekannte hämolytische Fähigkeit besitzt,

(b) des Inkubierens jeder Lösung während eines Zeitraums, der ausreicht zu ermöglichen, daß jegliches ASO in der Probe mit dem SO reagiert,

(c) des Wiederherstellens der hämolytischen Wirksamkeit des SO durch Einstellen des pH jeder inkubierten Lösung auf einen pH innerhalb des Bereiches von 4,8 bis 9,0, wodurch jedes freie SO im Überschuß zu dem Anteil, der zum Reagieren mit dem ASO erforderlich ist, in der Probe vorhandene Blutzellen auflösen kann, und

(d) des Feststellens der An- oder Abwesenheit von Auflösung in jeder Lösung.

9. Verfahren, wie in Anspruch 8 beansprucht, worin in Schritt (b) jede Lösung bei einer Temperatur von 0 bis 50°C inkubiert wird.

10. Verfahren, wie in Anspruch 8 oder 9 beansprucht, worin Schritt (a) weiter umfaßt

(1) das Herstellen zumindest einer Lösung von SO mit einem pH außerhalb des Bereiches von 4,8 bis 9,0 und das Mischen eines Volumens jeder SO-Lösung mit einer Blut- oder Serum-probe, wodurch der pH der gemischten Lösung außerhalb des Bereiches von 4,8 bis 9,0 bleibt, oder

(2) das Herstellen zumindest einer getrockneten Probe von SO, wobei das SO einen pH in Lösung außerhalb des Bereiches von 4,8 bis 9,0 aufweist, und das Mischen jeder Probe von SO mit einer nicht-hämolysierten Blut- oder Serumprobe in einer nicht-hämolytischen Lösung, wodurch der pH der gemischten Lösung außerhalb des Bereiches von 4,8 bis 9,0 bleibt, oder

(3) das Verdünnen der Blut- oder Serumprobe in einer nicht-hämolytischen Lösung, das Einführen von gleichen Volumina oder abgestuften Volumina der Blut- oder Serumprobelösung in jede einer Reihe von Testkammern, und das Zusetzen und Einmischen gleicher Konzentrationen oder abgestufter Konzentrationen von durch pH hämolytisch inaktiviertem SO in jede der die Blut- oder Serumproben enthaltenden Testkammern, wodurch der pH der gemischten Lösung in jeder Testkammer außerhalb des Bereiches von 4,8 bis 9,0 bleibt, oder

(4) des Einführen gleicher Konzentrationen oder abgestufter Konzentrationen von durch pH hämolytisch inaktiviertem, getrocknetem SO in jede einer Reihe von Testkammern, das Verdünnen der Blut- oder Serumprobe in einer nicht-hämolytischen Lösung, und das Zusetzen und Einmischen von gleichen Volumina oder abgestuften Volumina der nicht-hämolytischen Blut- oder Serumprobelösung in jede der das SO enthaltenden Testkammern, wodurch der pH der gemischten Lösung in jeder Testkammer außerhalb des Bereiches von 4,8 bis 9,0 bleibt.

11. Verfahren, wie in einem der Ansprüche 8 bis 10 beansprucht, worin

(a) das Mischen der Blut- oder Serumprobe mit dem SO bei einem pH von mindestens 9,4 durchgeführt wird,

(b) jede gemischte Lösung bei einer Temperatur im Bereich zwischen Raumtemperatur und 50°C inkubiert wird, und

(c) jede inkubierte Lösung auf einen pH innerhalb des Bereiches von 6 bis 8 eingestellt wird.

12. Verfahren zum Messen des Antistreptolysin O (ASO)-Titers in einer Probe von Blut oder Serum, umfassend die Schritte

(a) des Verdünnens einer nicht-hämolysierten Blut- oder Serumprobe mit einer nicht-hämolytischen Lösung,

(b) das Einführens eines gleichen Volumens der verdünnten Blut- oder Serumprobe in eine Vielzahl von Testkammern, wobei jede der Testkammern eine getrocknete Probe von hämolytisch inaktivierten SO in abgestuften Anteilen enthält, wobei das SO eine vorherbestimmte hämolytische Fähigkeit aufweist und in Lösung einen pH von zumindest 9,4 vorsieht,

(c) des Mischens jeder Probe von verdünntem Blut oder Serum mit dem SO,

(d) des Inkubierens jeder gemischten Lösung bei einer Temperatur im Bereich von Raumtemperatur bis 50°C während 1 bis 10 min,

(e) des Einstellens des pH jeder inkubierten Lösung auf einen pH im Bereich von 6 bis 8 und

(f) des Feststellens der An- oder Abwesenheit von Auflösung in jeder inkubierten Lösung 5 bis 25 min nach Einstellen des pH jeder Lösung.

13. Verfahren zum Messen des Antistreptolysin O (ASO)-Titers in einer Probe von Blut oder Serum, umfassend die Schritte

(a) des serienweisen Verdünnens einer nicht-hämolysierten Blut- oder Serumprobe mit einer nicht-hämolytischen Lösung,

(b) des Einführens gleicher Volumina der serienweise verdünnten Blut- oder Serumprobe in eine Vielzahl von Testkammern, wobei jede der Testkammern eine getrocknete Probe von hämolytisch inaktiviertem SO in gleichen Anteilen enthält, wobei das genannte SO eine vorherbestimmte hämolytische Fähigkeit aufweist und in Lösung einen pH von zumindest 9,4 ergibt,

(c) des Mischens jeder Probe des serienweise verdünnten Bluts oder Serums mit den SO,

(d) des Inkubierens jeder gemischten Lösung bei einer Temperatur im Bereich von Raumtemperatur bis 50°C während 1 bis 10 min,

(e) des Einstellens des pH jeder inkubierten Lösung auf einen pH im Bereich von 6 bis 8 und

(f) des Feststellens der An- oder Abwesenheit von Auflösung in jeder inkubierten Lösung 2 bis 20 min nach Einstellen des pH jeder Lösung.

14. Methode zum Beobachten des Verlaufes eines Krankheitszustandes oder einer Infektion, der bzw. die von einer Infektion von Streptococcus der Gruppe A stammt, umfassend

(1) das Messen des Antistreptolysin O (ASO)-Titers in Blutproben, die vom gleichen Individuum zu verschiedenen Zeiten während des Verlaufs des Krankheitszustandes oder der Infektion genommen wurden, nach einem Verfahren, wie in einem der Ansprüche 8 und 13 beansprucht, und

(2) das Bestimmen von Änderungen des ASO-Titers vom Individuum aus aufeinanderfolgenden Messungen,

wobei gemessene Zunahmen des ASO-Titers das Fortschreiten des genannten Krankheitszustandes oder der Infektion anzeigen und gemessene Abnahmen des ASO-Titers den Rückgang oder die Heilung des genannten Krankheitszustandes oder der Infektion anzeigen.

**Revendications**

1. Composition de réactif contenant de la streptolysine O réversiblement hémolytiquement inactive à utiliser pour la mesure de la quantité d'antistreptolysine O dans un échantillon, laquelle composition comprend une solution de streptolysine O et un acide, une base ou un tampon en une quantité suffisante pour maintenir le pH de la composition supérieur à 9,0 ou inférieur à 4,8.

2. Composition suivant la revendication 1, ayant un pH supérieur à 9,5 ou inférieur à 4,6.

3. Composition suivant la revendication 1, dans laquelle le pH est d'au moins 9,5.

4. Composition de réactif sèche contenant de la streptolysine O réversiblement hémolytiquement inactive à utiliser pour la mesure de la quantité d'antistreptolysine O dans un échantillon, qui comprend de la streptolysine O sous forme sèche et un acide, une base ou un tampon en une quantité suffisante pour maintenir le pH de la composition supérieur à 9,0 ou inférieur à 4,8 lorsque la composition sèche a été reconstituée avec de l'eau.

5. Composition suivant la revendication 4, dans laquelle la quantité de base ou de tampon est suffisante pour maintenir le pH de la composition à au moins 9,5.

6. Procédé de préparation d'un réactif à la streptolysine O réversiblement hémolytiquement inactive à utiliser pour la mesure de la quantité d'antistreptolysine O dans un échantillon, qui comprend le mélange de la streptolysine O avec un acide, une base ou un tampon en une quantité suffisante pour maintenir le pH supérieur à 9,0 ou inférieur à 4,8.

7. Procédé suivant la revendication 6, dans lequel le mélange est séché pour donner le réactif sous forme sèche.

8. Procédé pour mesurer le titre en antistreptolysine O (ASLO) d'un échantillon de sang ou de sérum, qui comprend les stades

(a) de mélanger au moins un échantillon de sang ou de sérum non hémolysé avec au moins une quantité déterminée au préalable de streptolysine O (SLO) dans une solution non hémolytique ayant un pH supérieur à 9,0 ou inférieur à 4,8, cette SLO ayant une capacité hémolytique connue;

(b) d'incuber chaque solution pendant une durée suffisante pour permettre à l'ASLO éventuelle de l'échantillon de réagir avec la SLO;

(c) de rétablir l'activité hémolytique de la SLO par ajustement du pH de chaque solution incubée jusqu'à un pH dans le domaine de 4,8 à 9,0, de façon que la SLO libre éventuelle en excès sur la quantité nécessaire pour réagir avec l'ASLO puisse lyser les globules rouges présents dans l'échantillon, et

(d) de détecter la présence ou l'absence de lyse dans chaque solution.

9. Procédé suivant la revendication 8, dans lequel au stade (b), chaque solution est incubée à une température de 0 à 50°C.

10. Procédé suivant la revendication 8 ou 9, dans lequel le stade (a) comprend en outre

(1) la préparation d'au moins une solution de SLO ayant un pH en dehors du domaine de 4,8 à 9,0; et le mélange d'un volume de chaque solution de SLO avec un échantillon de sang ou de sérum, de façon que le pH de la solution mélangée reste en dehors du domaine de 4,8 à 9,0, ou

(2) la préparation d'au moins un échantillon séché de SLO, la SLO ayant un pH en solution en dehors du domaine de 4,8 à 9,0; et le mélange de chaque échantillon de SLO avec un échantillon de sang ou de sérum non hémolysé dans une solution non hémolytique, de façon que le pH de la solution mélangée reste en dehors du domaine de 4,8 à 9,0, ou

(3) la dilution de l'échantillon de sang ou de sérum dans une solution non hémolytique; l'introduction de volumes égaux ou de volumes gradués de la solution d'échantillon de sang ou de sérum dans chacune d'une série de chambres de test; et l'apport et le mélange, dans chacune des chambres de test contenant les échantillons de sang ou de sérum, de concentrations égales ou de concentrations graduées en SLO hémolytiquement inactivée par le pH, de façon que le pH de la solution mélangée dans chaque chambre de test reste en dehors du domaine de 4,8 à 9,0, ou

(4) l'introduction de concentrations égales ou de concentrations graduées de SLO séchée hémolytiquement inactivée par le pH dans chacune d'une série de chambres de test; la dilution de l'échantillon de sérum ou de sang dans une solution non hémolytique; et l'apport et le mélange, dans chacune des chambres de test contenant la SLO, de volumes égaux ou de volumes gradués de la solution d'échantillon de sang ou de sérum non hémolytique, de façon que le pH de la solution mélangée dans chaque chambre de test reste en dehors du domaine de 4,8 à 9,0.

11. Procédé suivant l'une quelconque des revendications 8 à 10, dans lequel

(a) le mélange de l'échantillon de sang ou de sérum avec la SLO est exécuté à un pH d'au moins 9,4;

(b) chaque solution mélangée est incubée à une température de l'intervalle de la température ambiante jusqu'à 50°C; et

(c) chaque solution incubée est ajustée jusqu'à un pH dans le domaine de 6 à 8.

12. Procédé pour mesurer le titre en antistreptolysine O (ASLO) d'un échantillon de sérum ou de sang, comprenant les stades

(a) de diluer un échantillon de sang ou de sérum non hémolysé avec une solution non hémolytique;

(b) d'introduire un volume égal de l'échantillon de sang ou de sérum dilué dans plusieurs chambres de test, chacune des chambres de test contenant un échantillon séché de SLO hémolytiquement inactivée en quantités graduées, la SLO ayant une capacité hémolytique déterminée au préalable et ayant en solution un pH d'au moins 9,4;

(c) de mélanger chaque échantillon de sang ou de sérum dilué avec la SLO;

(d) d'incuber chaque solution mélangée à une température s'échelonnant de la température ambiante à 50°C pendant 1 à 10 minutes;

(e) d'ajuster le pH de chaque solution incubée jusqu'à un pH s'échelonnant de 6 à 8; et

(f) de détecter la présence ou l'absence de lyse dans chaque solution incubée 5 à 25 minutes après l'ajustement du pH de chaque solution.

13. Procédé pour mesurer le titre en antistreptolysine O (ASLO) dans un échantillon de sang ou de sérum, comprenant les stades

(a) de diluer en sérieur un échantillon de sang ou de sérum non hémolysé avec une solution non hémolytique;

(b) d'introduire des volumes égaux de l'échantillon de sang ou de sérum dilué en série dans plusieurs chambres de test, chacune des chambres de test contenant un échantillon séché de SLO hémolytiquement inactivée en quantités égales, la SLO ayant une capacité hémolytique déterminée au préalable et ayant en solution un pH d'au moins 9,4;

(c) de mélanger chaque échantillon du sang ou du sérum diluée en série avec la SLO;

(d) d'incuber chaque solution mélangée à une température s'échelonnant de la température ambiante à 50°C pendant 1 à 10 minutes;

(e) d'ajuster le pH de chaque solution incubée à un pH s'échelonnant de 6 à 8; et

(f) de détecter la présence ou l'absence de lyse dans chaque solution incubée 2 à 20 minutes après l'ajustement du pH de chaque solution.

14. Procédé pour surveiller l'évolution d'un état pathologique ou d'une infection résultant d'une atteinte par un streptocoque du groupe A, comprenant

(1) la mesure, par un procédé suivant l'une quelconque des revendications 8 et 13, du titre en antistreptolysine O (ASLO) d'échantillons du sang prélevés sur le même sujet à différents moments pendant l'évolution de l'état pathologique ou de l'infection, et

(2) la détermination, à partir des mesures successives, des changements du titre en ASLO du sujet,

les augmentations mesurées du titre en ASLO indiquant une progression de l'état pathologique ou de l'infection, et

les diminutions mesurées du titre en ASLO indiquant une régression ou la guérison de l'état pathologique ou de l'infection.